Europäisches Patentamt

European Patent Office

Office européen des brevets

(11) Publication number: **0 311 392 B1**

## EUROPEAN PATENT SPECIFICATION

(45) Date of publication of patent specification: **29.12.93** (51) Int. Cl.5: **C07K 1/02**, C07K 7/06

(21) Application number: **88309307.2**

(22) Date of filing: **06.10.88**

The file contains technical information submitted after the application was filed and not included in this specification

(54) **Solution synthesis of an octapeptide.**

(30) Priority: **07.10.87 GB 8723485**

(43) Date of publication of application:
**12.04.89 Bulletin 89/15**

(45) Publication of the grant of the patent:
**29.12.93 Bulletin 93/52**

(84) Designated Contracting States:
**AT BE CH DE ES FR GB GR IT LI LU NL SE**

(56) References cited:

**No further relevant documents found than the cited reference in this application.**

(73) Proprietor: **FARMITALIA CARLO ERBA S.r.L.**
**Via Carlo Imbonati 24**
**I-20159 Milano(IT)**

(72) Inventor: **de Castiglione, Roberto**
**Via Domenichino, 38**
**Milan(IT)**
Inventor: **Galantino, Mauro**
**Via dei Carracci, 8**
**Milan(IT)**
Inventor: **Forino, Romualdo**
**Via del Caravaggio, 14**
**Milan(IT)**

(74) Representative: **Woods, Geoffrey Corlett et al**
**J.A. KEMP & CO.**
**14 South Square**
**Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 311 392 B1

## Description

The present invention relates to a method for the preparation of the octapeptide of formula H-Leu-Glu-Asp-Gly-Pro-Lys-Phe-Leu-OH (A) and its pharmaceutically acceptable salts. The peptide, having thymic humoral activity was originally isolated from calf thymus glands and then obtained by solid-phase synthesis as described in US-A-4,621,135.

The present synthesis, based on conventional solution methods, offers the advantage of easier scale-up and better final yields for the purified target product. As a matter of fact, in the process of the present invention there is no formation of a succinimidyl derivative which is the main cyclic byproduct formed in the solid-phase synthesis of the prior art. The byproduct is formed by an internal reaction between the $\beta$-carboxy group of Asp (even if protected) and the amino group of Gly. This reaction occurs in strongly acidic or basic conditions, such as those employed in the synthesis of US-A-4,621,135 for removing the final peptide from the solid support with hydrofluoric acid.

The synthetic scheme according to the invention can be represented as follows:

```
        OBzl    OBzl
         |       |
Z——Leu——Glu——Asp——Gly——Pro——OTce
                 |
                 ↓

     OBzl OBzl                              W
      |    |                                |
Z——Leu——Glu——Asp——Gly——Pro——OH    +    H——Lys——Phe——Leu——Q
        (B)                                        (C)
                          ↓

     OBzl OBzl                    W
      |    |                      |
Z——Leu——Glu——Asp——Gly——Pro——Lys——Phe——Leu——Q
              (D)
                    ↓

H——Leu——Glu——Asp——Gly——Pro——Lys——Phe——Leu——OH
              (A)
```

wherein:

W represents an amino protecting group, Z is benzyloxycarbonyl, Q represents a carboxy protecting group, Bzl is benzyl and Tce is 2,2,2-trichloroethyl. The peptides (B) and (C) are condensed, the resultant peptide (D) is deprotected and, if desired, the resulting peptide (A) is converted into a pharmaceutically acceptable salt.

The groups W, Z, Q and Bzl are protecting groups which block amino and carbonyl groups not involved in the formation of the peptide linkage. These protecting groups are removable by acidolysis, saponification, hydrogenolysis or other methods according to the methods known in peptide chemistry. The following groups may be the amino protecting group W: benzyloxycarbonyl t-butyloxycarbonyl, trityl, formyl, trifluoroacetyl, o-nitrophenylsulphenyl, 4-methoxybenzyloxycarbonyl, 9-fluorenylmethoxycarbonyl, 3,5-dimethoxy-$\alpha,\alpha'$dimethylbenzyloxycarbonyl and methylsulphonylethoxycarbonyl. The following groups may be used for the carboxy protecting group Q: methyl, ethyl, t-butyl, benzyl, p-nitrobenzyl, 9-fluorenylmethyl, phenyl, 2,2,2-trichloroethyl.

The condensation between the amino group of peptide (C) and the carboxy group of peptide (B) to form the intermediate (D) may be carried out through an activated acyl-derivative such as a mixed anhydride, an azide, an activated ester, an imidazole or substituted imidazole derivative or by direct condensation between the free amino group and the carboxyl group, in the presence of a condensing agent such as dicyclohexyl carbodiimide alone or together with N-hydroxysuccinimide or 1-hydroxybenzotriazole or 1-hydroxybenzotriazole and 4-dimethylamino-pyridine. The activated acyl-derivative may be formed "in situ" starting from the compound (B) using known methods.

The condensation may be carried out in a solvent such as dimethyl formamide, pyridine, acetonitrile, tetrahydrofuran, N-methyl-2-pyrrolidone and dimethylsulfoxide. The reaction temperature may be from -30°C to ambient temperature. The reaction time is generally from 1 to 120 hours.

The protecting groups and the condensing agents are selected so as to avoid the risk of racemization. The peptide of formula (A) or pharmaceutically acceptable salt thereof thus produced is recovered. It may be formulated with a pharmaceutically acceptable carrier or diluent to prepare a pharmaceutical composition for administration.

The preparation of starting materials (B) and (C) can be accomplished by known classical solution methods.

The following Example illustrates the invention. A Reference Example is also provided. Rf values were determined on pre-coated plates of silica gel 60 $F_{254}$ (Merck), layer thickness 0.25 mm, length 20 cm, using the following development systems:

System A: n-butanol/acetic acid/water = 4/1/1 by volume.

System B: benzene/ethyl acetate/acetic acid/water = 10/10/2/1 by volume (upper phase).

"Merck" is a Trade Mark.

TLC analyses were carried out at a temperature ranging from 18°C to 25°C: the Rf values therefore can change by ± 5%. The Rt values were obtained from HPLC analyses using the following systems:

System 1 - Mobile phase: A (0.02 M $KH_2PO_4$, pH 3.5, buffer containing 10% v/v acetonitrile); B (0.02 M $KH_2PO_4$, pH 3.5, buffer containing 70% v/v acetonitrile)

Column: $\mu$ BONDAPAK ™ $C_{18}$ (WATERS) 10 $\mu$m (3.9 mm x 30 cm)

Flow rate: 1 ml/min

U.V. detection: 210 nm.

System 2 - Mobile phase: A (0.02 M $KH_2PO_4$, pH 3.5, buffer containing 10% v/v acetonitrile); B (0.02 M $KH_2PO_4$, pH 3.5, buffer containing 70% v/v acetonitrile)

column: RP-18 5 $\mu$m (4 mm x 25 cm) Lichrosorb (Merck)

Flow rate: 1 ml/min

U.V. detection: 210 nm.

System 3 - Mobile phase: A (0.02 M $KH_2PO_4$, pH 3.3, buffer containing 10% v/v acetonitrile); B (0.02 M $KH_2PO_4$, pH 3.3, buffer containing 60% v/v acetonitrile)

Column: $\mu$ BONDAPAK ™ $C_{18}$ (WATERS) 10 $\mu$m (3.9 mm X 30 cm)

Flow rate: 1.5 ml/min

U.V. detection: 210 nm

Melting points were determined in open capillaries with a Tottoli apparatus and are uncorrected.

Most of derivatives soften and decompose before melting.

In this specification symbols and abbreviations are those commonly used in peptide chemistry (see, e.g., Eur. J. Biochem., 138, 9-37 (1984)).

Other symbols and abbreviations used in the following Reference Example and Example are: AcOH, glacial acetic acid; AcOEt, ethyl acetate; dec., decomposition; DMF, dimethyl formamide; ECC, ethyl chloroformate; $Et_2O$, diethyl ether; HPLC, high performance liquid chromatography; MeOH, methyl alcohol; NMM, N-methyl morpholine; p-Tos-OH, para-toluensulphonic acid; THF, tetrahydrofuran; TLC, thin layer chromatography, Tce 2,2,2,trichloroethyl, DCC dicyclohexyl carbodiimide, HOBt, 1-hydroxybenzotriazole, DMAP 4-dimethylaminopyridine, DCEU, dicyclohexylurea.

## REFERENCE EXAMPLE

### Preparation of HCl. H-Lys(Z)-Phe-Leu-OBzl (V)

### Step 1 - p-Tos-OH . H-Leu-OBzl (I)

To a suspension of 13.12 g (100 mmoles) of H-Leu-OH in 105 ml of benzyl alcohol and 200 ml of anhydrous chloroform, 38.0 g (200 mmoles) of p-Tos-OH . $H_2O$ were added and the mixture was refluxed for 25 h. The water produced (5.4 ml) was collected using a Dean and Stark separator designed for use with solvents denser than water. Trituration with 500 ml of $Et_2O$ after cooling and evaporation of the chloroform gave 37.46 g (95% yield) of compound (I): m.p. 124-126 °C; $[\alpha]_D^{20}$ = -3.0° (C 1, MeOH); $Rf_A$ = 0.65; $Rt_1$ = 16.7 minutes (linear gradient from 10 to 60% B in 20').

### Step 2 - Boc-Phe-Leu-OBzl (II)

To a solution of 41.52 g (156,5 mmoles) of Boc-Phe-OH in 240 ml of anhydrous THF, 17.36 ml (156.5 mmoles) of NMM and 15.36 ml (156.5 mmoles) of ECC were successively added at a temperature of -12 °C. After stirring at this temperature for 2 minutes, a cold solution of 61.52 g (156.5 mmoles) of p-Tos-OH . H-Leu-OBzl (I) and 17.36 ml (156.5 mmoles) of NMM in 480 ml of DMF was added. The reaction mixture was stirred for 1 hour at -12 °C, then filtered from salts and evaporated in vacuo. The residue was dissolved in AcOEt and washed several times successively with NaCl saturated solutions of 0.5 M citric acid, 0.5 M NaHCO$_3$ and water. The organic layer was dried over anhydrous Na$_2$SO$_4$ and the solvent removed in vacuo.

69 g (94.1% yield) of compound (II) were obtained from AcOEt/light petroleum: m.p. 84-87 °C; $[\alpha]_D^{20}$ = - 22.0° (C 1, MeOH); Rf$_B$ = 0.70; Rt$_3$ = 11.0 minutes (isocratic 90% B for 15')

### Step 3 - HCl . H-Phe-Leu-OBzl (III)

32.0 g (68.3 mmoles) of Boc-Phe-Leu-OBzl (II) were dissolved in 320 ml of a saturated solution of HCl in AcOH. After 30 minutes at room temperature the Boc-removal was complete and the solvent was evaporated in vacuo at 30 °C.

26.0 g (94% yield) of compound (III) were obtained from AcOEt: m.p. = 158-160 °C; $[\alpha]_D^{20}$ = -17.5° (C 1, MeOH); Rf$_A$ = 0.72; Rt$_2$ = 13.8 minutes (linear gradient from 60 to 90% B in 20').

### Step 4 - Boc-Lys(Z)-Phe-Leu-OBzl (IV)

Starting from 24.37 g (64.1 mmoles) of HCl . H-Phe-Leu-OBzl (III), and operating as in Step 2, 42.01 g (89.7% yield) of compound (IV) were obtained from AcOEt/Et$_2$O: m.p. 120-123 °C; $[\alpha]_D^{20}$ = -28.8° (C 1, MeOH); Rf$_B$ = 0.62; Rt$_2$ = 21.0 minutes (isocratic 90% B for 30').

### Step 5 - HCl . H-Lys(Z)-Phe-Leu-OBzl (V)

Starting from 41.96 g (57.4 mmoles) of Boc-Lys(Z)-Phe-Leu-OBzl (IV) and operating as described in Step 3, 36.82 g (96.1% yield) of compound (V) were obtained from MeOH/AcOEt: m.p. = 187-191 °C; $[\alpha]_D^{20}$ = -6.3° (C 1, MeOH); Rf$_A$ = 0.61; Rt$_2$ = 11.0 minutes (isocratic 90% B for 20')

### EXAMPLE

Preparation of H-Leu-Glu-Asp-Gly-Pro-Lys-Phe-Leu-OH. 2HCl by DCC/HOBt coupling

### Step 1: Z-Leu-Glu(OBzl)-Asp(OBzl)-Gly-Pro-OH (VI)

A solution of Z-Leu-Glu(OBzl)-Asp(OBzl)-Gly-Pro-OTce in AcOH and water was made to react at room temperature with zinc powder, washed with HCl 0.1 N, acetone and Et$_2$O. After filtration of the reaction mixture on hyflo supercell, the solvent was evaporated, the residue was dissolved in AcOEt and washed several times successively with NaCl saturated solutions of 0.5 M citric acid and water. The organic layer was dried over anhydrous Na$_2$SO$_4$, the solvent removed in vacuo, and the compound (VI) was obtained. Rf$_B$ = 0.20.

### Step 2: Z-Leu-Glu(OBzl)-Asp(OBzl)-Gly-Pro-Lys(Z)-Phe-Leu-OBzl (VII)

A solution of Z-Leu-Glu(OBzl)-Asp(OBzl)-Gly-Pro-OH (VI) and HCl.H-Lys(Z)-Phe-Leu-OBzl (V) in DMF, were made to react for 4 hours at room temperature with DCC, anhydrous HOBt, DMAP and NMM. The reaction mixture was filtered from DCEU, concentrated to a small volume, and the compound (VII) was obtained, m.p. 179-182°C (dec.)

### Step 3: H-Leu-Glu-Asp-Gly-Pro-Lys-Phe-Leu-OH (VIII)

A solution of Z-Leu-Glu(OBzl)-Asp(OBzl)-Gly-Pro-Lys(Z)-Phe-Leu-OBzl (VII) in AcOH and MeOH was made to react for 20 minutes at room temperature with 10% palladium on charcoal, formic acid and NMM (N-methyl-morpholine).

After filtration of the reaction mixture on hyflo supercell, the solvent was evaporated and the compound (VIII) was obtained from EtOH/AcOEt, m.p. 206-210°C (dec.)

Step 4: 2HCl.H-Leu-Glu-Asp-Gly-Pro-Lys-Phe-Leu-OH (IX)

A solution of compound (VIII) in HCl 1N was evaporated to residue. The title compound (IX) was obtained from AcOH/Et$_2$O: m.p. = 105°C (softening) - 160°C (dec.); $[\alpha]_D^{20}$ = -58.4° (C 0.5 MeOH); Rt$_1$ = 12.0 minutes (linear gradient from 10 to 60% B in 20').

**Claims**

1. A process for preparing a peptide of the formula (A):

$$\text{H---Leu---Glu---Asp---Gly---Pro---Lys---Phe---Leu---OH} \qquad \text{(A)}$$

or a pharmaceutically acceptable salt thereof, which process comprises treating with zinc powder the compound of formula:

$$
\begin{array}{c}
\text{OBzl} \quad\; \text{OBzl} \\
| \qquad\quad\; | \\
\text{Z---Leu---Glu---Asp---Gly---Pro---OTce}
\end{array}
$$

wherein Z is benzyloxycarbonyl, Bzl is benzyl and Tce is 2,2,2-trichloroethyl, thereby obtaining the compound of the formula (B):

$$
\begin{array}{c}
\text{OBzl} \quad\; \text{OBzl} \\
| \qquad\quad\; | \\
\text{Z---Leu---Glu---Asp---Gly---Pro---OH} \qquad \text{(B)}
\end{array}
$$

wherein Z and Bzl are as defined above; condensing said compound of the formula (B) with a compound of formula (C)

$$
\begin{array}{c}
\text{W} \\
| \\
\text{H---Lys---Phe---Leu---Q} \qquad\qquad\qquad\qquad\qquad \text{(C)}
\end{array}
$$

wherein W is an amino protecting group and Q represents a carboxy protecting group; deprotecting the resultant compound of the formula (D)

$$
\begin{array}{c}
\text{OBzl} \; \text{OBzl} \qquad\qquad\qquad\quad \text{W} \\
| \qquad\; | \qquad\qquad\qquad\qquad\; | \\
\text{Z---Leu---Glu---Asp---Gly---Pro---Lys---Phe---Leu---Q} \qquad \text{(D)}
\end{array}
$$

wherein Z, Bzl, W and Q are as defined above; and, if desired, converting the resulting peptide of formula (A) into a pharmaceutically acceptable salt thereof.

**2.** A process according to claim 1, further comprising formulating the peptide of formula (A) or pharmaceutically acceptable salt thereof thus produced with a pharmaceutically acceptable carrier or diluent.

**Patentansprüche**

**1.** Verfahren zum Herstellen eines Peptids der Formel (A):

$$H—Leu—Glu—Asp—Gly—Pro—Lys—Phe—Leu—OH \qquad (A)$$

oder eines pharmazeutisch geeigneten Salzes davon, umfassend Behandeln der Verbindung der Formel:

$$\begin{array}{cc} OBzl & OBzl \\ | & | \\ Z—Leu—Glu—Asp—Gly—Pro—OTce \end{array}$$

wobei Z Benzyloxycarbonyl ist, Bzl Benzyl ist und Tce 2,2,2-Trichlorethyl ist, mit Zinkpulver unter Erhalt der Verbindung der Formel (B)

$$\begin{array}{cc} OBzl & OBzl \\ | & | \\ Z—Leu—Glu—Asp—Gly—Pro—OH \end{array} \qquad (B)$$

worin Z und Bzl wie zuvor angegeben definiert sind; Kondensieren der Verbindung der Formel (B) mit einer Verbindung der Formel (C)

$$\begin{array}{c} W \\ | \\ H—Lys—Phe—Leu—Q \end{array} \qquad (C)$$

wobei W eine Amino schützende Gruppe ist und Q eine Carboxy schützende Gruppe darstellt; Entfernen der Schutzgruppen der sich ergebenden Verbindung der Formel (D)

$$\begin{array}{ccc} OBzl & OBzl & W \\ | & | & | \\ Z—Leu—Glu—Asp—Gly—Pro—Lys—Phe—Leu—Q \end{array} \qquad (D)$$

worin Z, Bzl, W und Q wie zuvor angegebenen definiert sind, und gewünschtenfalls Umwandeln des sich ergebenden Peptids der Formel (A) in ein pharmazeutisch geeignetes Salz davon.

**2.** Verfahren nach Anspruch 1, welches weiterhin Formulieren des auf diese Weise hergestellten Peptids der Formel (A) oder eines pharmazeutisch geeigneten Salzes davon mit einem pharmazeutisch geeigneten Träger oder Verdünnungsmittel umfaßt.

**Revendications**

**1.** Procédé pour préparer un peptide de formule (A) : H - Leu- Glu -Asp - Gly - Pro - Lys - Phe - Leu - OH (A) ou l'un de ses sels pharmaceutiquement acceptables, procédé qui comprend le traitement avec de la poudre de zinc le composé de formule :

$$\underset{Z}{\overset{}{}} \text{---Leu---} \underset{\overset{|}{OBzl}}{Glu} \text{---} \underset{\overset{|}{OBzl}}{Asp} \text{---Gly ---Pro---OTce}$$

dans laquelle Z est le radical benzyloxycarbonyle, Bzl est le radical benzyle et Tce est le radical 2,2,2-trichloroéthyle, de façon à obtenir le composé de formule (B) :

$$Z \text{--- Leu ---} \underset{\overset{|}{OBzl}}{Glu} \text{---} \underset{\overset{|}{OBzl}}{Asp} \text{--- Gly --- Pro --- OH} \qquad (B)$$

dans laquelle Z et Bzl sont tels que définis ci-dessus, la condensation du composé de formule (B) avec un composé de formule (C)

$$H \text{--- } \underset{\overset{|}{W}}{Lys} \text{---Phe --- Leu --- Q} \qquad (C)$$

dans laquelle W est un groupe amino-protecteur et Q représente un groupe carboxy-protecteur ; la déprotection du composé obtenu de formule (D)

$$Z \text{--- Leu ---} \underset{\overset{|}{OBzl}}{Glu} \text{---} \underset{\overset{|}{OBzl}}{Asp} \text{--- Gly ---Pro ---} \underset{\overset{|}{W}}{Lys} \text{---Phe --- Leu --- Q} \ (D)$$

dans laquelle Z, Bzl, W et Q sont tels que définis ci-dessus; et si on le souhaite, la conversion du peptide obtenu de formule (A) en l'un de ses sels pharmaceutiquement acceptables.

2.  Procédé selon la revendication 1, comprenant en outre la formulation du peptide de formule (A) ou l'un de ses sels pharmaceutiquement acceptables ainsi produits, avec un support ou un diluant pharmaceutiquement acceptable.